# EUROPEAN PATENT APPLICATION

(11) **EP 4 548 832 A1**
(43) Date of publication of application: **07.05.2025**
(21) Application number: 24209793.9
(22) Date of filing: 30.10.2024
(51) Int. Cl.: A61B 3/00

(54) **CHIN REST DEVICE AND OPHTHALMOLOGIC APPARATUS**

(30) Priority: 01.11.2023 JP 2023187941; 06.02.2024 JP 2024016591
(71) Applicant: TOPCON CORPORATION, Tokyo 174-8580 (JP)
(72) Inventor: INUZUKA, Naoki, Tokyo, 174-8580 (JP)
(74) Representative: Louis Pöhlau Lohrentz

(57) **Abstract**

A chin rest device (18, 18A, 18B, 18C, 18D, 18E), wherein the chin rest device (18, 18A, 18B, 18C, 18D, 18E) is configured to be detachably attached to an optical table (12) or a pedestal, the optical table (12) of an ophthalmologic apparatus (100) is configured to acquire information about a subject eye (E), and the pedestal (30) is configured to support a measurement head (16, 31) for acquiring information about the subject eye (E) such that the measurement head (16, 31) is movable in horizontal and vertical directions. The chin rest device (18, 18A, 18B, 18C, 18D, 18E) comprises an attachment (181) that is configured to be detachably attached to the optical table (12) or the pedestal (30); and a chin rest portion (182) that is configured to extend from the attachment (181) toward a chin (C) of an examinee (P) to support the chin (C).

## Description

### FIELD OF THE INVENTION

The present invention relates to a chin rest device and an ophthalmologic apparatus.

### BACKGROUND

It is known in the art that an ophthalmologic apparatus includes a measurement head, which includes a measurement optical system for acquiring information about subject eyes and a visual-target presenting portion for presenting visual targets to the subject eyes and is suspended in front of the subject eyes by a supporting portion (see JP2019-216817A and JP2022-064300A). It is also known that an ophthalmologic apparatus includes a refractor head having test lenses, which is suspended by a supporting portion and positioned in front of the subject eyes, while the apparatus presents visual targets to the subject eyes and acquires information about the subject eyes (see JP2023-048902A).

Each of the ophthalmologic apparatuses disclosed in JP2019-216817A, JP2022-064300A, and JP2023-048902A includes a forehead rest for supporting the forehead of an examinee to prevent his or her face from accidentally moving when acquiring information about the subject eyes. Further, the ophthalmologic apparatus disclosed in JP2022-064300A includes a cheek contact portion with two cheek-contact bodies for supporting the cheeks of an examinee. The cheek contact portion and the forehead contact portion support the face of the examinee, thereby improving the face support stability. JP2018-175011 has disclosed a head-mounted display-type ophthalmologic apparatus including a chin rest for supporting the chin of an examinee and fixed to a table that supports the elbows of the examinee. JP2015-058107 has disclosed an ophthalmologic apparatus that includes a pedestal; a measurement head, which includes a measurement optical system for acquiring information about the subject eyes and which is supported on the pedestal to be movable relative to the subject eyes in horizontal and vertical directions; and a chin rest portion. These conventional ophthalmologic apparatuses allow the examinee to undergo the examinations in a comfortable posture by supporting the examinee's face, thereby acquiring information about the subject eyes more appropriately and quickly. However, there is still a strong need for developing technology that allows the examinee to undergo the examinations more comfortably and appropriately.

The present invention has been made in view of the above circumstances. An object of the present invention is to acquire information about the subject eyes more appropriately while providing the examinee with greater comfort.

### SUMMARY

According to one embodiment of the present invention, a chin rest device that is configured to be detachably attached to an optical table or a pedestal, the optical table of an ophthalmologic apparatus is configured to acquire information about a subject eye, the pedestal is configured to support a measurement head for acquiring information about the subject eye such that the measurement head is movable in horizontal and vertical directions. The chin rest device includes an attachment that is configured to be detachably attached to the optical table or the pedestal; and a chin rest portion that is configured to extend from the attachment toward a chin of an examinee to support the chin.

According to another embodiment of the present invention, an ophthalmologic apparatus includes a measurement head including a measurement optical system that is configured to acquire information about a subject eye; an optical table that is configured to support the measurement head; and the above chin rest device. The chin rest device is configured to be detachably attached to the optical table.

According to yet another embodiment of the present invention, an ophthalmologic apparatus includes a pedestal; a measurement head that includes a measurement optical system for acquiring information about a subject eye, the measurement head being supported on the pedestal such that the measurement head is movable relative to the subject eye in horizontal and vertical directions; and the above chin rest device. The chin rest device is configured to be detachably attached to the pedestal.

The chin rest device with the above configuration can support the face of the examinee more stably, allowing the examinee to receive examinations in a comfortable posture while enabling the acquisition of information about the subject eyes to be more accurate and efficient. The user can also easily attach and detach the chin rest device as needed. Therefore, it is possible to acquire information about the subject eyes more accurately while providing greater comfort for the examinee.

### BRIEF DESCRIPTION OF THE DRAWINGS

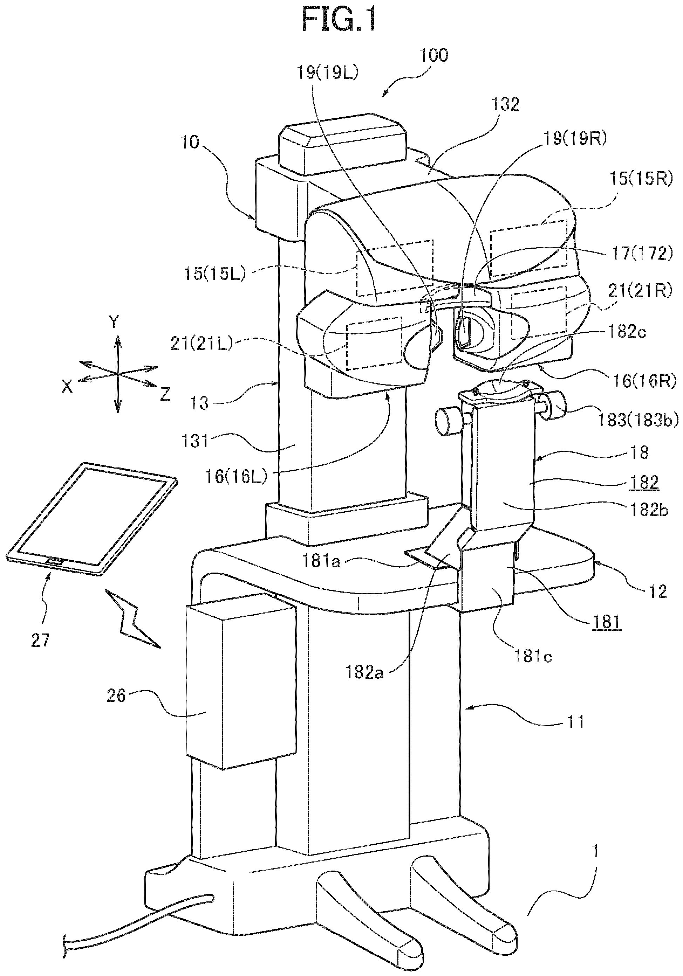

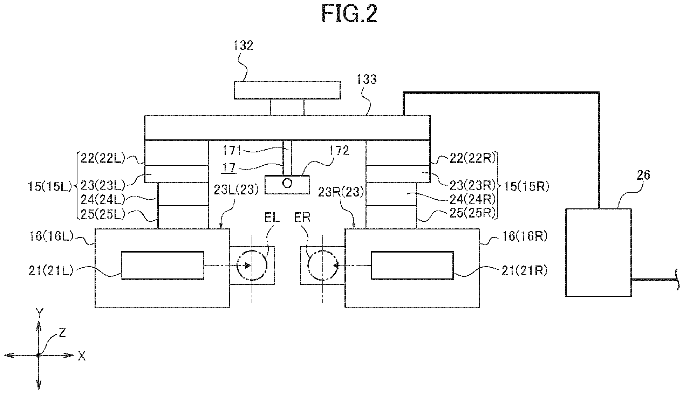

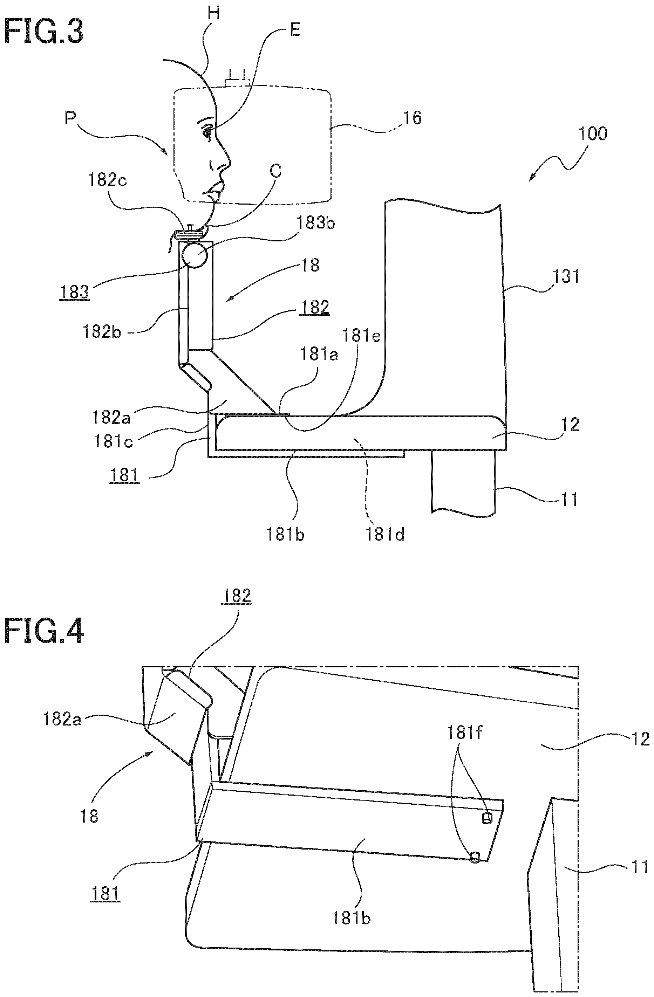

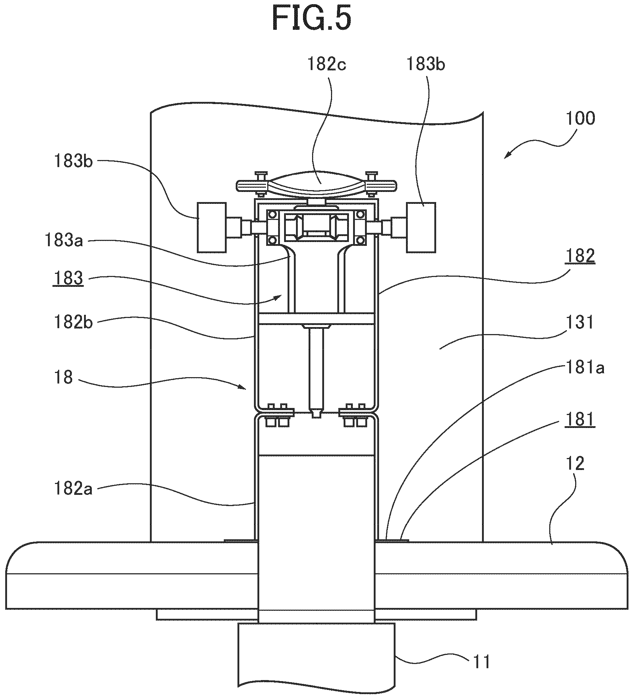

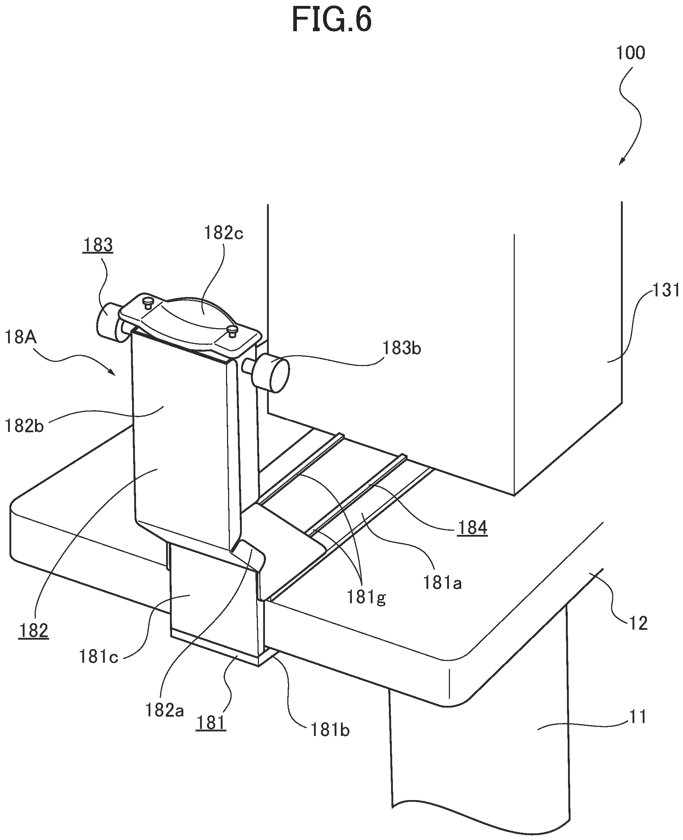

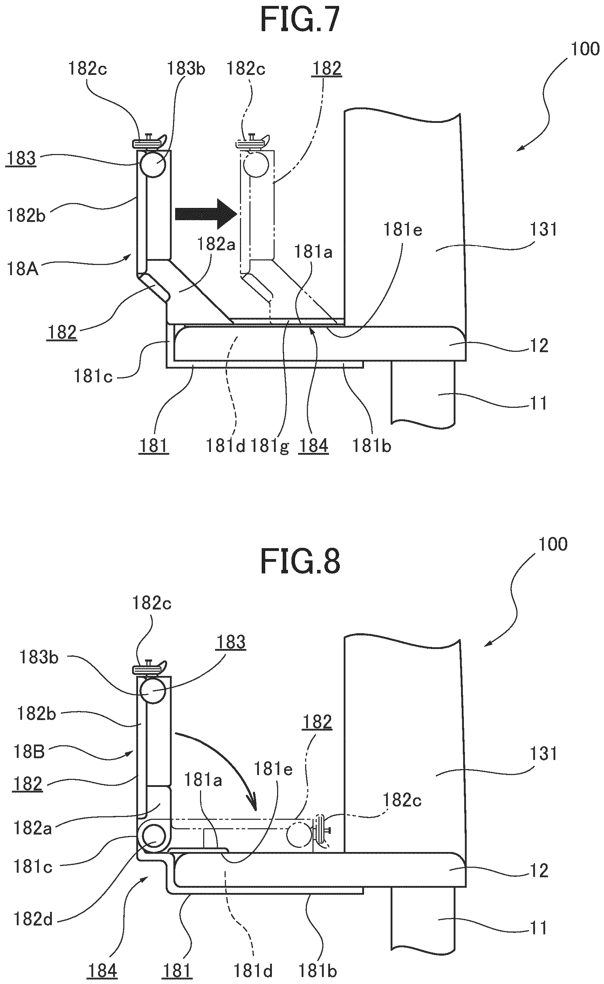

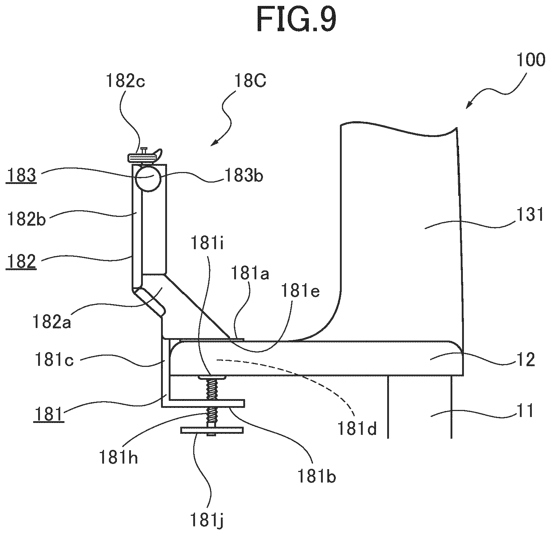

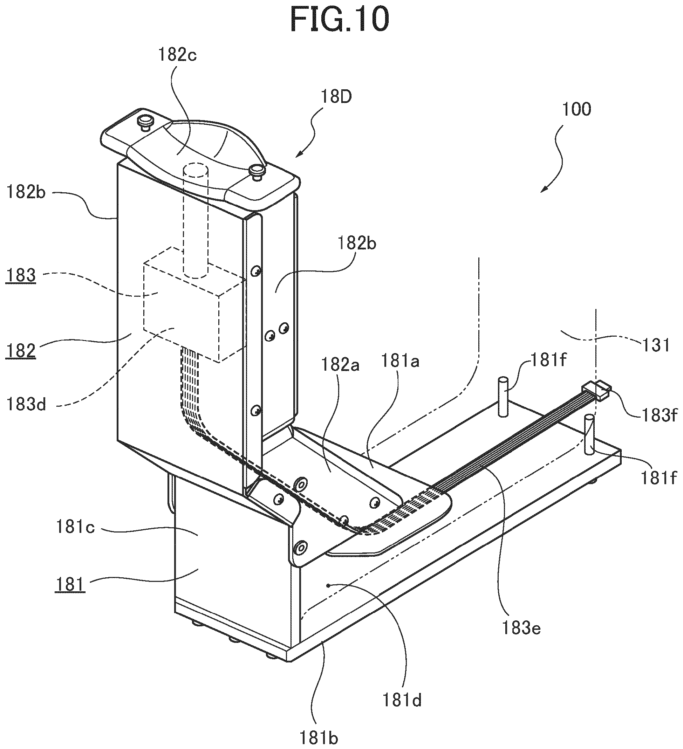

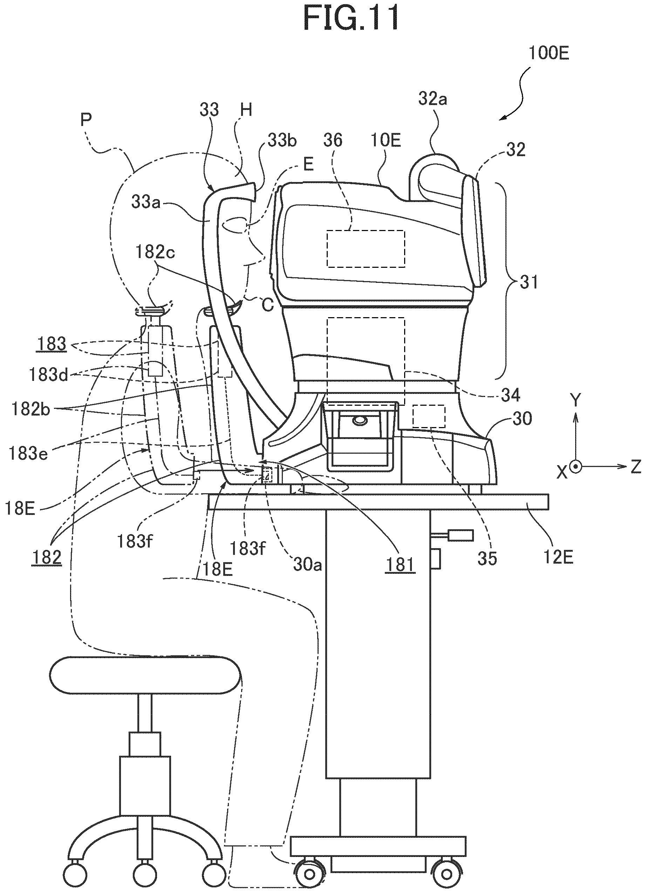

FIG. 1 is a perspective view showing the entire structure of an ophthalmologic apparatus according to a first embodiment. FIG. 2 is a schematic view showing a major part of the ophthalmologic apparatus according to the first embodiment. FIG. 3 is a side view showing a part including a chin rest device of the ophthalmologic apparatus according to the first embodiment. FIG. 4 is a perspective view showing the underside of the chin rest device of the ophthalmologic apparatus according to the first embodiment. FIG. 5 is a schematic view showing an inner structure of the chin rest device of the ophthalmologic apparatus according to the first embodiment. FIG. 6 is a perspective view showing a part including a chin rest device of an ophthalmologic apparatus according to a second embodiment. FIG. 7 is a side view showing a part including the chin rest device of the ophthalmologic apparatus according to the second embodiment. FIG. 8 is a side view showing a part including a chin rest device of an ophthalmologic apparatus according to a third embodiment. FIG. 9 is a side view showing a part including a chin rest device of an ophthalmologic apparatus according to a fourth embodiment. FIG. 10 is a perspective view showing a chin rest device of an ophthalmologic apparatus according to a fifth embodiment. FIG. 11 is a side view showing an ophthalmologic apparatus according to a sixth embodiment.

### DETAILED DESCRIPTION

With respect to the use of plural and/or singular terms herein, those having skill in the art can translate from the plural to the singular and/or from the singular to the plural as is appropriate to the context and/or application. The various singular/plural permutations may be expressly set forth herein for the sake of clarity.

### First Embodiment

An ophthalmologic apparatus 100 according to the first embodiment of the present invention is described with reference to FIGS. 1 to 5. The ophthalmologic apparatus 100 of the first embodiment is a binocular open-type ophthalmologic apparatus capable of simultaneously acquiring information of left and right subject eyes E (also referred to as "ocular information" hereinafter) when an examinee P opens his or her left and right subject eyes E (see FIG. 3, etc.). The ophthalmologic apparatus 100 of the first embodiment also can acquire the ocular information of each subject eye E at a time by shielding one of the eyes or turning off a fixed target for one of the subject eyes E.

The ophthalmologic apparatus 100 of the first embodiment is configured to enable both objective and subjective examinations. In the subjective examination, the ophthalmologic apparatus 100 presents a visual target at a predetermined position to the examinee P and acquires subjective examination results as ocular information based on the responses from the examinee P to the visual target. The subjective examination includes subjective refraction tests such as the far-point test, the intermediate-point test, the near-point test, the contrast test, the glare test, the pinhole test, the stereo vision test, and the visual field test. In the objective examination, the ophthalmologic apparatus 100 projects light onto the subject eyes E and measures the ocular information based on the detection results of the reflected light. The objective examinations include measurements to obtain characteristics of the subject eyes E as the ocular information and imaging to capture images of the subject eyes E. Additionally, the objective examinations include the objective refractive measurement (refraction measurement), the corneal shape measurement (keratometry measurement), the intraocular pressure measurement, the ocular fundus imaging, the tomogram imaging using optical coherence tomography (OCT) (OCT imaging), and other measurements using OCT.

(Overall Configuration of Ophthalmologic Apparatus) The ophthalmologic apparatus 100 of the first embodiment mainly includes a body portion 10, a chin rest device 18, and an examiner's control device 27. The ophthalmologic apparatus 100 may also include other components and/or devices, such as an examinee's control device not shown in the drawings. As shown in FIG. 1, the body portion 10 includes a base 11, an optical table 12, a head supporting portion 13, drive mechanisms 15, a pair of measurement heads 16 (16L, 16R), a forehead rest 17, and a controller 26.

The head supporting portion 13 supports the pair of the measurement heads 16 by suspending them. The head supporting portion 13 also supports the forehead rest 17, suspending it between the measurement heads 16. The chin rest device 18 is detachably attached to the optical table 12.

The ophthalmologic apparatus 100 of the first embodiment is configured to acquire information about the subject eyes E of the examinee P when the examinee P is positioned relative to the optical table 12 with his or her forehead placed on the forehead rest 17, which is suspended between the measurement heads 16, and his or her chin C placed on the chin rest device 18, which is attached to the optical table 12. As shown in FIG. 1, the present specification defines an X-axis, a Y-axis, and a Z-axis. From the perspective of the examinee P, the left and right direction is defined as the X-direction, the vertical direction is defined as the Y-direction, and the direction that is orthogonal to both the X- and Y-directions and gets closer to and away from the examinee P (the depth direction of the measurement heads 16, which is the front-back direction) is defined as the Z-direction. In the Z-direction, the direction towards the examinee P is defined as the forward direction, and the direction away from the examinee P is defined as the rearward direction.

The base 11 is placed on a floor 1 and supports the optical table 12. The optical table 12 is configured to hold the examiner's control device 27, the examinee's control device (not shown), and tools used in optometry. The optical table 12 may also serve as support for the examinee P to place his or her hands. The base 11 supports the optical table 12 such that the position in the Y-direction (height direction) of the optical table 12 is adjustable via a known drive mechanism such as an electric motor.

As shown in FIGS. 1 and 2, the head supporting portion 13 includes a column 131, an arm portion 132, and an attachment base portion 133. The base 11 supports the column 131 such that the column 131 extends in the Y-direction at the rear end of the optical table 12.

The arm portion 132 is provided at the distal end of the column 131. The arm portion 132 is configured to suspend both measurement heads 16 above the optical table 12 via the drive mechanism 15. The arm portion 132 extends from the column 131 towards the front (Z-direction) and includes the attachment base portion 133 at its distal end. The arm portion 132 is movable in the Y-direction relative to the column 131. The arm portion 132 may also be movable in the X-direction and Z-direction relative to the column 131. The attachment base portion 133 at the end of the arm portion 132 suspends a pair of the drive mechanisms 15, and each of which suspends the corresponding measurement head 16. In other words, the head supporting portion 13 supports a pair of the measurement heads 16 via a pair of the drive mechanisms 15.

The measurement heads 16 are provided as a pair, corresponding respectively to the left and right subject eyes E of the examinee. Hereinafter, when described individually, the measurement heads 16 are referred to as the left-eye measurement head 16L and the right-eye measurement head 16R. The left-eye measurement head 16L acquires information about the left subject eye E (EL) of the examinee P, while the right-eye measurement head 16R acquires information about the right subject eye E (ER) of the examinee P. The left-eye and right-eye measurement heads 16L and 16R are symmetrically arranged with respect to a vertical plane positioned in the middle between them in the X-direction.

As shown in FIG. 1, each of the measurement heads 16 is provided with a mirror 19 (referred to as a left-eye mirror 19L or a right-eye mirror 19R when mentioned individually) as a deflecting member, and houses a measurement optical system 21 (referred to as a left-eye measurement optical system 21L or a right-eye measurement optical system 21R when mentioned individually). Each of the measurement heads 16 acquires information about the corresponding subject eye E with the measurement optical system 21 via the mirror 19.

Each measurement optical system 21 includes various optical systems such as a Z alignment system, an XY alignment system, a keratometry measurement system, a visual-target projection system (visual-target presenting portion), an anterior ocular segment observation system with an image acquisition portion, a refraction measurement projection system, and a refraction measurement light receiving system, all of which are constructed using various optical elements. A measurement optical system disclosed in, for example, JP2019-216817A may be used as the measurement optical system 21, but it is not limited to this.

Each of the measurement optical systems 21 consists of, or combines, one or more of the following devices: a visual acuity testing device that conducts visual acuity tests while switching the visual targets to be presented; a phoropter that acquires the appropriate corrective refractive power for the subject eye E by switching corrective lenses; a refractometer or a wavefront sensor that measures refractive power; a fundus camera that captures images of the fundus of the eye; a tomographic device that takes tomographic images of the retina, a specular microscope that captures corneal endothelium images; a keratometer that measures the corneal shape; and a tonometer that measures intraocular pressure.

The drive mechanisms 15 include a left-eye drive mechanism 15L corresponding to the left-eye measurement head 16L, and a right-eye drive mechanism 15R corresponding to the right-eye measurement head 16R. As shown in FIG. 2, the left-eye drive mechanism 15L includes a vertical drive portion 22L for the left eye, a horizontal drive portion 23L for the left eye, an X-direction rotation drive portion (horizontal rotation drive portion) 24L for the left eye, and a Y-direction rotation drive portion (vertical rotation drive portion) 25L for the left eye. As shown in FIG. 2, the right-eye drive mechanism 15R includes a vertical drive portion 22R for the right eye, a horizontal drive portion 23R for the right eye, an X-direction rotation drive portion (horizontal rotation drive portion) 24R for the right eye, and a Y-direction rotation drive portion (vertical rotation drive portion) 25R for the right eye.

The drive portions 22L to 25L corresponding to the left-eye measurement head 16L and the drive portions 22R to 25R corresponding to the right-eye measurement head 16R are symmetrically arranged with respect to the vertical plane positioned in the middle between the left-eye and right-eye measurement heads 16L and 16R in the X direction. Hereinafter, these drive portions may be collectively referred to as vertical drive portion(s) 22, horizontal drive portion(s) 23, X-direction rotation drive portion(s) 24, and Y-direction rotation drive portion(s) 25 unless otherwise individually described. The same applies to other components that are symmetrically provided on the left and right.

The drive mechanism 15 is formed with the vertical drive portion 22, the horizontal drive portion 23, the X-direction rotation drive portion 24, and the Y-direction rotation drive portion 25 arranged in that order from the upper side of the drive mechanism 15.

The vertical drive portions 22 are fixed to the attachment base portion 133 at the distal end of the arm portion 132 and are configured to move the horizontal drive portions 23, the X-direction rotation drive portions 24, and the Y-direction rotation drive portions 25 in the Y direction (vertical direction) relative to the arm portion 132 in response to a control signal from the controller 26. The horizontal drive portions 23 are fixed to the vertical drive portions 22 and are configured to move the X-direction rotation drive portions 24 and the Y-direction rotation drive portions 25 in the X and Z directions (horizontal direction) relative to the vertical drive portions 22 in response to the control signal from the controller 26.

Each of the vertical drive portions 22 and the horizontal drive portions 23 includes an actuator, such as a pulse motor, to produce a driving force, and a transmission mechanism, such as a combination of gears or a rack and pinion, to transmit the driving force. Each of the horizontal drive portions 23 is designed to facilitate easy configuration and control of the movement in the horizontal direction, for example, by individually providing a combination of actuators and transmission mechanisms for the X and Z directions, respectively.

The X-direction rotation drive portions 24 are connected to the horizontal drive portions 23. In response to a control signal from the controller 26, the X-direction rotation drive portions 24 rotate the corresponding measurement heads 16 and the Y-direction rotation drive portions 25 relative to the horizontal drive portions 23 in the X direction (horizontal direction) about respective vertical eyeball rotation axes. The vertical eyeball rotation axes extend in the vertical direction (Y direction) through the eyeball rotation points of the corresponding subject eyes E.

The Y-direction rotation drive portions 25 are connected to the X-direction rotation drive portions 24 and suspend the measurement heads 16. In response to a control signal from the controller 26, the Y-direction rotation drive portions 25 rotate the corresponding measurement heads 16 relative to the X-direction rotation drive portions 24 in the Y direction (vertical direction, top-bottom direction) about a pair of horizontal eyeball rotation axes (rotation axes). The horizontal eyeball rotation axes extend in the horizontal direction (X direction) through the eyeball rotation points of the corresponding subject eyes E.

The X-direction rotation drive portions 24 and the Y-direction rotation drive portions 25 may be configured so that, for example, the transmission mechanisms receiving the driving forces from the actuators move along arc-shaped guide grooves. The central position of each of the guide grooves is aligned with the pair of horizontal ocular rotation axes and the pair of vertical ocular rotation axes, respectively. Thus, the X-direction rotation drive portions 24 and the Y-direction rotation drive portions 25 rotate the measurement heads 16 about the pair of horizontal eyeball rotation axes and the pair of vertical eyeball rotation axes of the subject eyes E.

The X-direction rotation drive portions 24 may be configured to support the Y-direction rotation drive portions 25 and the measurement heads 16 so that they are rotatable about the rotation axes thereof, and to rotate the measurement heads 16 while adjusting the positions where the measurement heads 16 are supported, in cooperation with the horizontal drive portions 23. In addition, the Y-direction rotation drive portions 25 may be configured to support the measurement heads 16 so that they are rotatable about the rotation axes thereof, and to rotate the measurement heads 16 while adjusting the positions where the measurement heads 16 are supported, in cooperation with the vertical drive portions 22.

With the above configuration, the drive mechanisms 15 can move the measurement heads 16 individually or in conjunction in the X, Y, and Z directions, and can rotate the measurement heads 16 about the vertical eyeball rotation axes and the horizontal eyeball rotation axes of the subject eyes E in the X and Y directions. The controller 26 of the ophthalmologic apparatus 100 in the first embodiment controls each of the drive portions 22 to 25 of the drive mechanism 15, thereby moving and rotating each measurement head 16. Furthermore, the examiner, as the user, may manually operate each of the drive portions 22 to 25 to move and rotate each of the measurement heads 16.

In addition, the X-direction rotation drive portion 24L for the left eye and the X-direction rotation drive portion 24R for the right eye rotate the left-eye measurement head 16L and the right-eye measurement head 16R in the X direction (left-right direction), respectively, allowing for divergence (divergence movement) or convergence (convergence movement) of the subject eyes E. Furthermore, the Y-direction rotation drive portion 25L for the left eye and the Y-direction rotation drive portion 25R for the right eye rotate the left-eye measurement head 16L and the right-eye measurement head 16R in the Y direction (vertical direction), respectively, directing the lines of sight of the subject eyes E downward and/or returning the lines to their original positions. Thus, the ophthalmologic apparatus 100 can conduct tests for both divergence and convergence movements, as well as tests at various distances, ranging from a far-point test at a far-point distance to a near-point test at a near-point distance with the binocular vision to measure the ocular information of both subject eyes E.

The forehead rest 17 is configured to support the forehead H of the examinee P and to stabilize the face of the examinee P in place to prevent any changes in its orientation and position. The forehead rest 17 is suspended from the attachment base portion 133 between the pair of measurement heads 16. As shown in FIGS. 1 and 2, the forehead rest 17 includes a forehead support rod 171 and a forehead rest body 172. The forehead support rod 171 is a rod-shaped member that is attached to the attachment base portion 133 and extends downward and forward therefrom. The forehead rest body 172 is attached to the lower end of the forehead support rod 171 and is positioned above the pair of measurement heads 16. The forehead rest body 172 has an elongated oval shape with a curved surface to fit the forehead H of the examinee P. Moving the arm portion 132 relative to the column 131 in the Y direction adjusts the vertical position of the forehead rest 17.

The chin rest device 18 is configured to support the chin C of the examinee P and to stabilize the face of the examinee P in place to prevent any changes in its orientation and position. The ophthalmologic apparatus 100 of the first embodiment supports the face of the examinee P with both the chin rest device 18 and the forehead rest 17, thereby improving the stability of the face of the examinee P.

As mentioned above, the chin rest device 18 is detachably attached to the optical table 12. Accordingly, a provider of the ophthalmologic apparatus 100, such as a sales company, can provide the ophthalmologic apparatus 100 with the chin rest device 18 attached to the optical table 12 for users who need it, and can also provide the ophthalmologic apparatus without the chin rest device 18 for users who do not need it. Additionally, the provider can supply the user with just the chin rest device 18, and the user can attach the chin rest device 18 to an existing ophthalmologic apparatus.

In the ophthalmologic apparatus 100 of the first embodiment, the user can attach and detach the chin rest device 18 on their own. Thus, considering factors such as the type of examination or test, the condition of the examinee P, and the preferences of the examinee P, the user can use the ophthalmologic apparatus 100 with or without the chin rest device 18, as desired.

The chin rest device 18 mainly includes an attachment 181, a chin rest portion 182, and a vertical-position adjusting mechanism 183. Details of each part of the chin rest device 18 are described hereinafter with reference to FIGS. 3 to 5.

The attachment 181 is detachably attached to the optical table 12. The attachment 181 includes an upper support member 181a that is disposed on the upper surface of the optical table 12, a lower support member 181b that is disposed on the lower surface of the optical table 12, and a connecting portion 181c that connects the first ends of the upper and lower support members 181a and 181b. When viewed from the side, the attachment 181 has a C-shape with the second ends of the upper and lower support members 181a and 181b being open. The attachment 181 includes an attachment space 181d that is defined by the upper and lower support members 181a and 181b and the connecting portion 181c, along with an opening 181e that is accessible from the second ends of the upper and lower support members 181a and 181b. The height of the attachment space 181d is substantially equal to the thickness of the optical table 12. The front side of the optical table 12 is inserted into the attachment space 181d through the opening 181e and is then sandwiched between the upper and lower support members 181a and 181b.

The upper support member 181a is positioned near the front end of the upper surface of the optical table 12. The lower support member 181b is longer in the rearward direction (Z direction) than the upper support member 181a and is fastened to the optical table 12 near the far side (column 131) using a pair of left and right bolts 181f. Thus, the chin rest device 18 is stably fixed to the optical table 12 by the bottom surface of the upper support member 181a and the pair of left and right bolts 181f, preventing any accidental displacement or detachment of the chin rest device 18.

The chin rest portion 182 is configured to support the chin C and extends upward from the upper support member 181a of the attachment 181 toward the chin C of the examinee P. As shown in FIG. 3, the chin rest portion 182 of the first embodiment includes a base portion 182a, a rectangular housing 182b, and a chin receiving platform 182c. The base portion 182a extends from the upper support member 181a in a forward, oblique upward direction. The rectangular housing 182b rises vertically from the base portion 182a. The chin receiving platform 182c extends from the housing 182b and is vertically movable. The chin rest portion 182 has a V shape when viewed from the side. This V-shaped extension of the chin rest portion 182 reduces the depth of the optical table 12 and provides more space in front of the examinee P, thereby preventing the optical table 12 and the chin rest device 18 from interfering during the examinations.

In the first embodiment and other embodiments, the components such as each part of the attachment 181, and the base portion 182a and the housing 182b of the chin rest portion 182 are made from metal plates for ease of processing and low cost. However, they are not limited to metal plates and may also be made of resin, wood, or a combination of these materials.

The chin receiving platform 182c is configured to support the chin C of the examinee P. The chin receiving platform 182c includes a horizontally elongated plate member with a downwardly concave curved surface and a rod member that is movable upward and downward.

The vertical-position adjusting mechanism 183 is configured to adjust the vertical position or height of the chin receiving platform 182c. The overall position of the chin rest device 18 can be adjusted by moving the optical table 12 in the Y direction. However, the distance between the forehead H and the chin C of the examinee P varies from person to person. Accordingly, the chin rest device 18 of the first embodiment is capable of adjusting the vertical position (height) of the chin receiving platform 182c relative to the optical table 12 using the vertical-position adjusting mechanism 183.

As shown in FIG. 5, the vertical-position adjusting mechanism 183 is located within the housing 182b of the chin rest portion 182. The vertical-position adjusting mechanism 183 includes a known movement mechanism 183a, such as a ball spline or rack-and-pinion, and height adjusting knobs 183b that project from both sides of the housing 182b. The movement mechanism 183a is configured to move the chin receiving platform 182c upward and downward based on the direction and degree of the rotation of the height adjusting knobs 183b. More specifically, for example, rotating the height adjusting knobs 183b clockwise activates the movement mechanism 183a to move the chin receiving platform 182c upward, while rotating the height adjusting knobs 183b counterclockwise activates the movement mechanism 183a to move the chin receiving platform 182c downward.

The chin rest device 18 is directly attached to the optical table 12, separate from the measurement heads 16 that are suspended from the head supporting portion 13. This configuration ensures that the vertical movement of the measurement heads 16 does not affect the position of the chin rest device 18. The position of the chin rest device 18 can be freely adjusted in the vertical direction using the vertical-position adjusting mechanism 183, according to the position of the chin C, independent of the measurement heads 16.

The examiner's control device 27 is an information processing device configured to receive input operations (e.g., input of examinee's ID, examination items, parameters, and instructions) by the examiner and to output control signals to the controller 26. The examiner's control device 27 may consist of a device such as a smartphone or a tablet terminal that includes, for example, a controller (CPU), a monitor, an input section, a microphone, and a speaker. Additionally, the examiner's control device 27 may consist of a laptop personal computer, a desktop personal computer, or a dedicated controller provided in the ophthalmologic apparatus 100. The examiner's control device 27 is configured to communicate with the controller 26 via communication means such as short-distance wireless communication or the Internet.

The examinee's control device, which may be included in the ophthalmologic apparatus 100, is a device used by the examinee P to respond during the acquisition of information regarding the subject eyes E. The examinee's control device may include, for example, a keyboard, a mouse, a joystick, a touchpad, and a touch panel. The examinee's control device is connected to the controller 26 via a wired or wireless communication channel and outputs control signals to the controller 26 based on the response operations input into the examinee's control device.

The controller 26 is installed on the base 11 of the body portion 10 and comprehensively controls each part of the ophthalmologic apparatus 100. The controller 26 is connected to components such as the measurement optical system 21, the drive mechanism 15, the examiner's control device 27, and the examinee's control device, allowing it to control them all comprehensively.

The controller 26 may consist of a processor such as a CPU. The controller 26 is configured to comprehensively control the operation of the ophthalmologic apparatus 100 based on operations made through the examiner's control device 27 and the examinee's control device by loading a program stored in a memory such as a storage device connected to the controller 26 or built-in ROM, for example, onto the RAM.

The controller 26 acquires ocular information based on input data such as examination items, parameters, and/or instructions provided by the examiner's control device 27. The examination items include both objective and subjective examinations as mentioned above. The parameters are information necessary for the examinations, such as initial values for visual acuity and corrected power, the visual targets to be presented, and the examination distance.

The operation of the examiner and the examinee P in acquiring ocular information using the ophthalmologic apparatus 100 of the first embodiment with the aforementioned configuration is described as follows. Suppose that the ophthalmologic apparatus 100 has been powered on and that the controller 26 can communicate with both the examiner's control device 27 and the examinee's control device.

During the examination, the examinee P sits on a chair and faces the ophthalmologic apparatus 100. At this time, the examiner may adjust the positions (height positions) of the chin rest device 18 and the measurement heads 16, along with the optical table 12, in the Y direction by moving the optical table 12 vertically according to the physical attributes of the examinee P such as the height. Then, the examinee P applies his or her forehead to the forehead rest body 172 of the forehead rest 17. Next, the examiner rotates the height adjusting knobs 183b of the vertical-position adjusting mechanism 183 to operate the movement mechanism 183a, which moves the chin receiving platform 182c vertically. This operation positions the chin receiving platform 182c to receive the chin C of the examinee P, thereby supporting the chin C of the examinee P with the chin receiving platform 182c.

Accordingly, the examinee P can undergo the examinations in a comfortable posture, with his or her face properly fixed by the forehead rest 17 and the chin rest device 18, without needing to strain his or her neck or support the body with his or her arms. After the examinee P's face is properly fixed, the examiner operates the examiner's control device 27 to command the body portion 10 to execute the operation for acquiring ocular information. First, the ophthalmologic apparatus 100 of the first embodiment performs the alignment process. During the alignment process, the measurement head 16 moves vertically and horizontally and rotates via the drive mechanism 15 according to the position of the subject eyes E; however, the chin rest device 18 remains stationary in both the vertical and rotational directions, thereby providing stable support for the chin C.

After completing the alignment, the ophthalmologic apparatus 100 executes the aforementioned objective and subjective examinations of the subject eyes E based on the examiner's instructions or automatically. Even during these examinations, the chin rest device 18 effectively suppresses any accidental movement of the face. Accordingly, even if the examinee P responds to inquiries about visibility during both the objective and subjective examinations, the face remains stable, allowing the examinee P to undergo the examinations in a comfortable posture and helping to prevent the need for retesting. This is especially beneficial for examinees P with relatively low physical strength, such as the elderly or children, as the chin C is continuously supported by the chin rest device 18 even during prolonged examinations. This ensures that the face does not move accidentally, helps reduce fatigue, and allows the examinee to undergo the examination more comfortably. Consequently, each examination can be performed more effectively and efficiently.

### Second Embodiment

An ophthalmologic apparatus 100 according to the second embodiment of the present invention is described as follows with reference to FIGS. 6 and 7. The ophthalmologic apparatus 100 of the second embodiment has the same basic configuration as the ophthalmologic apparatus 100 of the first embodiment shown in FIGS. 1 to 5, except that it includes a chin rest device 18A, as shown in FIGS. 6 and 7, in place of the chin rest device 18. Accordingly, the same signs as in the first embodiment are assigned to the same parts, and their detailed descriptions are omitted. The same applies to other embodiments described later. In the following, the configuration of the chin rest device 18A of the second embodiment is mainly described. Similar to the chin rest device 18 of the first embodiment, the chin rest device 18A of the second embodiment, as well as the chin rest devices 18B, 18C, and 18D of the third to fifth embodiments, are detachably attached to the optical table 12.

As shown in FIGS. 6 and 7, the chin rest device 18A of the second embodiment includes an attachment 181, a chin rest portion 182, and a vertical-position adjusting mechanism 183. The vertical-position adjusting mechanism 183 is configured similarly to that in the first embodiment. The attachment 181 has a C-shape when viewed from the side and includes an upper support member 181a, a lower support member 181b, a connecting portion 181c, an attachment space 181d, and an opening 181e. The lower support member 181b is elongated in the Z direction, similar to that in the first embodiment, and is fixed to the optical table 12 by bolts 181f, 181f on its side close to the head supporting portion 13 (see FIG. 5).

The upper support member 181a is an elongated member that extends in the Z direction to the vicinity of the column 131 and includes a pair of rail members 181g on its top surface. The chin rest portion 182 of the second embodiment is positioned on the rail members 181g to be movable in the front-rear direction (Z direction). The pair of rail members 181g functions as a retraction mechanism 184 that moves and retracts the chin rest portion 182 to a retraction position W when the chin rest device 18 is not in use. Additionally, the pair of rail members 181g functions as a front-rear position movement mechanism that allows for the adjustment of the position of the chin rest portion 182 in the Z direction. The retraction position W of the second embodiment is a rear position on the pair of rail members 181g, located away from the examinee P and near the column 131. In another embodiment, a recess may be provided in the column 131 as a retraction position W to store the chin rest portion 182 upon retraction.

Similar to the first embodiment, the chin rest portion 182 includes a base portion 182a, a housing 182b, and a chin receiving platform 182c, forming a V shape when viewed from the side. The chin rest portion 182 includes moving elements such as wheels and sliding balls (not shown), which facilitate manual movement in the Z direction along the rail members 181g of the upper support member 181a.

In the ophthalmologic apparatus 100 of the second embodiment with the above configuration, the chin rest portion 182 is disposed at one end of the pair of rail members 181g on the side of the examinee P during the examination, as shown by the solid line in FIG. 7. The examinee P, who faces the ophthalmologic apparatus 100, applies his or her forehead to the forehead rest body 172 of the forehead rest 17. The examiner moves the optical table 12 in the vertical direction to adjust the position in the Y direction, as necessary. Furthermore, the examiner can also move the chin rest portion 182 in the front-rear direction according to the position of the face of the examinee P to adjust the position of the chin rest portion 182 in the Z direction.

Next, the examiner rotates the height adjusting knobs 183b to operate the movement mechanism 183a, which moves the chin receiving platform 182c in the vertical direction. This operation positions the chin receiving platform 182c to receive the chin C of the examinee P, thereby supporting the chin C on the chin receiving platform 182c. Accordingly, the ophthalmologic apparatus 100 of the second embodiment can stably fix the face of the examinee P using the forehead rest 17 and the chin rest device 18A, allowing the examinee P to undergo the examinations comfortably in a comfortable posture, thereby enabling more appropriate and efficient objective and subjective examinations.

Furthermore, the ophthalmologic apparatus 100 of the second embodiment includes the retraction mechanism 184, allowing it to perform the examinations without using the chin rest portion 182. In this case, as shown by the broken line in FIG. 7, the examiner moves the chin rest portion 182 to the retraction position W so that the examinee P can undergo the examinations with only the forehead rest 17 supporting his or her face. With the chin rest portion 182 retracted from the front of the optical table 12, the top surface of the optical table 12 becomes open, allowing the examinee P to place his or her hands or arms on the top surface of the optical table 12 during the examinations.

The examiner can decide whether or not to use the chin rest portion 182, for example, depending on the condition of the examinee P. More specifically, for example, if the examinee P is relatively physically strong enough to keep his or her face still on his or her own, the examinations can be conducted without the chin rest portion 182. On the other hand, for example, if the examinee P is relatively physically weak, such as an elderly person, and has difficulty maintaining the same posture for a long time, the examinations can be conducted using the chin rest portion 182. Furthermore, the decision to use the chin rest portion 182 can also depend on factors such as the type of examination. More specifically, securely fixing the examinee's face with the chin rest portion 182 during the objective examination allows the examinee P to undergo the objective examination more precisely. On the other hand, not using the chin rest portion 182 during the subjective examination does not restrict the movement of the chin C of the examinee P, thereby allowing the examinee P to give verbal responses more smoothly. Even in this case, the face of the examinee P is appropriately supported by the forehead rest 17. Accordingly, the examinee P can undergo the examinations more comfortably and effectively.

### Third Embodiment

An ophthalmologic apparatus 100 according to the third embodiment of the present invention is described as follows with reference to FIG. 8. The ophthalmologic apparatus 100 of the third embodiment includes the same basic configuration as the ophthalmologic apparatus 100 of the first embodiment shown in FIGS. 1 to 5, except that it includes a chin rest device 18B, shown in FIG. 8, in place of the chin rest device 18.

The chin rest device 18B of the third embodiment includes an attachment 181, a chin rest portion 182, and a vertical-position adjusting mechanism 183. The vertical-position adjusting mechanism 183 is configured similarly to that in the first embodiment. The attachment 181 has a C-shape when viewed from the side and includes an upper support member 181a, a lower support member 181b, a connecting portion 181c, an attachment space 181d, and an opening 181e. The lower support member 181b is elongated in the Z direction, similar to that in the first embodiment, and is fixed to the optical table 12 by bolts 181f on its side near the head supporting portion 13 (see FIG. 5).

Similar to the first embodiment, the chin rest portion 182 includes a base portion 182a, a housing 182b, and a chin receiving platform 182c, and has a V shape when viewed from the side. Furthermore, the lower end of the housing 182b of the chin rest portion 182 is rotatably supported on the base portion 182a by a shaft member 182d. The housing 182b, the base portion 182a, and the shaft member 182d function as a retraction mechanism 184 for the chin rest portion 182. The chin rest portion 182 can be rotated in the front-rear direction about the shaft member 182d, allowing it to be positioned either at a location where it can support the chin C of the examinee P, as shown by the solid line in FIG. 8, or at a retraction position W on the top surface of the optical table 12, as shown by the broken line in FIG. 8. The optical table 12 may also have a recess on the top surface as the retraction position W to store the chin rest portion 182 upon its retraction.

In the ophthalmologic apparatus 100 of the third embodiment, with the configuration described above, the chin rest portion 182 rises upward in the initial condition, as shown by the solid line in FIG. 8, to support the chin C of the examinee P. The examinee P, who faces the ophthalmologic apparatus 100, applies his or her forehead to the forehead rest body 172 of the forehead rest 17. The examiner moves the optical table 12 in the vertical direction to adjust the position in the Y direction, as necessary. Next, the examiner rotates the height adjusting knobs 183b to operate a movement mechanism 183a, which moves the chin receiving platform 182c in the vertical direction. This operation positions the chin receiving platform 182c to receive the chin C of the examinee P, thereby providing support for the chin C with the chin receiving platform 182c. Accordingly, the ophthalmologic apparatus 100 of the third embodiment can stably fix the face of the examinee P with the forehead rest 17 and the chin rest device 18B, allowing the examinee P to undergo the examinations more comfortably and in a better posture, thereby enabling the more appropriate and efficient objective and subjective examinations. Furthermore, the ophthalmologic apparatus 100 of the third embodiment includes the retraction mechanism 184, allowing the selection of whether or not to use the chin rest portion 182, depending on factors such as the condition of the examinee P and the type of the examination, similar to the second embodiment. Additionally, the chin rest portion 182 can be retracted more easily through the rotational movement.

### Fourth Embodiment

An ophthalmologic apparatus 100 according to the fourth embodiment of the present invention is described as follows with reference to FIG. 9. The ophthalmologic apparatus 100 of the fourth embodiment includes the same basic configuration as the ophthalmologic apparatus 100 of the first embodiment shown in FIGS. 1 to 5, except that it includes a chin rest device 18C, shown in FIG. 9, in place of the chin rest device 18.

The chin rest device 18C of the fourth embodiment includes an attachment 181, a chin rest portion 182, and a vertical-position adjusting mechanism 183. The chin rest portion 182 and the vertical-position adjusting mechanism 183 are configured similarly to those in the first embodiment. The attachment 181 of the fourth embodiment is a so-called clamp-type member. The attachment 181 includes an upper support member 181a, a lower support member 181b, a connecting portion 181c, an attachment space 181d, and an opening 181e, forming a C-shape when viewed from the side. The distance between the upper support member 181a and the lower support member 181b, that is, the height of the attachment space 181d, is greater than that of the attachment space 181d in the first embodiment, which allows the chin rest device 18C to be attached to the optical tables 12 with various thicknesses. The lower support member 181b includes a bolt 181h that is screwed into it. The bolt 181h includes a jaw 181i and a handle 181j. The jaw 181i is provided at the upper end of the bolt 181h, positioned inside the attachment space 181d and against the bottom surface of the optical table 12. The handle 181j is attached to the lower end of the bolt 181h, which extends outside the attachment space 181d.

The chin rest device 18C of the fourth embodiment is attached to the optical table 12, as follows. Firstly, the user, such as the examiner, inserts the optical table 12 into the attachment space 181d of the attachment 181 so that the upper support member 181a is positioned on the top surface of the optical table 12. Next, the user rotates the bolt 181h using the handle 181j to move the jaw 181i upward until it makes contact with the bottom surface of the optical table 12. Then, the user operates the handle 181j to clamp the optical table 12 between the upper support member 181a and the jaw 181i. Thus, the chin rest device 18C is securely attached to the optical table 12. The user can also easily detach the chin rest device 18C from the optical table 12 by reversing the operations described above.

The examinee P, facing the ophthalmologic apparatus 100 of the fourth embodiment with the above configuration, applies his or her forehead to the forehead rest body 172 of the forehead rest 17. The examiner then moves the optical table 12 in the vertical direction to adjust its position in the Y direction, as necessary. Next, the examiner rotates the height adjusting knobs 183b to operate the movement mechanism 183a, which moves the chin receiving platform 182c vertically. This operation positions the chin receiving platform 182c to receive the chin C of the examinee P, thereby providing support for the chin C with the chin receiving platform 182c. Consequently, the ophthalmologic apparatus 100 of the fourth embodiment can stably fix the face of the examinee P with the forehead rest 17 and the chin rest device 18C, allowing the examinee P to undergo the examinations more comfortably and maintain a suitable posture, thereby enabling more precise and efficient objective and subjective examinations. Since the chin rest device 18C of the fourth embodiment allows for the height adjustment of the jaw 181i, it can be attached to the optical tables 12 with various thicknesses, thus improving versatility. Considering factors such as the type of examination, the condition of the examinee P, and the preferences of the examinee P, the user can select whether or not to use the chin rest device 18C as necessary and can easily attach the chin rest device 18C to the optical table 12 and detach it therefrom based on the selection.

### Fifth Embodiment

An ophthalmologic apparatus 100 according to the fifth embodiment of the present invention will be described as follows with reference to FIG. 10. The ophthalmologic apparatus 100 of the fifth embodiment includes the same basic configuration as the ophthalmologic apparatus 100 of the first embodiment shown in FIGS. 1 to 5, except that it includes a chin rest device 18D shown in FIG. 10 in place of the chin rest device 18.

The chin rest device 18D of the fifth embodiment includes an attachment 181, a chin rest portion 182, and a vertical-position adjusting mechanism 183. The attachment 181 and the chin rest portion 182 have the same configurations as those in the first embodiment. The vertical-position adjusting mechanism 183 in the first to fourth embodiments allows for the adjustment of the height of the chin receiving platform 182c when the user, such as the examiner, manually rotates the height adjusting knobs 183b. In contrast, the vertical-position adjusting mechanism 183 of the fifth embodiment is configured to automatically adjust the height of the chin receiving platform 182c under the control of the controller 26. The examiner's control device 27 is configured to display the operation buttons for the vertical-position adjusting mechanism 183 on the operation screen of the monitor.

The vertical-position adjusting mechanism 183 of the fifth embodiment includes an electric drive portion 183d including elements such as an electric motor and an encoder, and a cable 183e. A terminal at the end of the cable 183e is connected to an electronic circuit board on which a CPU and other components are mounted to serve as the controller 26. The cable 183e transmits instruction signals from the controller 26 and electric power to the electric drive portion 183d.

The examinee P, who faces the ophthalmologic apparatus 100 of the fifth embodiment with the above configuration, applies his or her forehead to the forehead rest body 172 of the forehead rest 17. The examiner moves the optical table 12 vertically to adjust its position in the Y direction, as necessary. Next, the examiner operates the examiner's control device 27 to adjust the height of the chin rest device 18D. Based on the input of this operation, the controller 26 controls the electric drive portion 183d to move the chin receiving platform 182c vertically. This positions the chin receiving platform 182c to receive the chin C of the examinee P, thereby supporting the chin C with the chin receiving platform 182c. Consequently, the ophthalmologic apparatus 100 of the fifth embodiment can also stably fix the face of the examinee P with the forehead rest 17 and the chin rest device 18D, allowing the examinee P to undergo the examinations more comfortably in a suitable posture, thus enabling more appropriate and efficient objective and subjective examinations. The examiner can easily and accurately adjust the height of the chin rest portion 182 by operating the examiner's control device 27, even from a distance away from the ophthalmologic apparatus 100.

As a modified embodiment, the vertical-position adjusting mechanism 183, including the electric drive portion 183d of the fifth embodiment, may also be applied to the chin rest devices 18A, 18B, and 18C of the second to fourth embodiments. In the retraction mechanisms 184 of the chin rest devices 18A and 18B of the second and third embodiments, the chin rest portion 182 is manually moved or rotated in the front-rear direction. However, this is not limited to manual movement or rotation, and it may also be automatically moved or rotated under the control of the controller 26 using a known electric mechanism.

### Sixth Embodiment

An ophthalmologic apparatus 100E according to the sixth embodiment of the present invention is described below with reference to FIG. 11. As shown in FIG. 11, the ophthalmologic apparatus 100E of the sixth embodiment mainly includes a body portion 10E and a chin rest device 18E. The ophthalmologic apparatus 100E of the sixth embodiment is placed on an optical table 12E, which can be moved upward and downward using a known drive mechanism. However, the ophthalmologic apparatus 100E may also be placed on other types of desks, such as an office desk.

The body portion 10E includes a pedestal 30, a measurement head 31, a monitor portion 32, and a forehead rest 33. The pedestal 30 is placed on the optical table 12E. The measurement head 31 is supported on the pedestal 30 and is freely movable in the horizontal and vertical directions, that is, in the X, Y, and Z directions (hereinafter referred to as "XYZ directions"), relative to the subject eyes E.

As shown by the broken line in FIG. 11, the body portion 10E includes an XYZ drive mechanism 34 and a controller 35 therewithin. The XYZ drive mechanism 34 is configured to move the measurement head 31 in the XYZ directions using a known movement mechanism such as a stepper motor. The controller 35 includes one or more computer processors, which may be implemented using circuitry such as a CPU, and it comprehensively controls the operation of the ophthalmologic apparatus 100E. The controller 35 is electrically connected to the measurement optical system 36, the monitor portion 32, the XYZ drive mechanism 34 of the measurement head 31, and the chin rest device 18E and it controls their operations.

As shown by the broken line in FIG. 11, the measurement head 31 includes the measurement optical system 36 for observation and imaging. The measurement optical system 36 includes optical components, such as optical lenses, imaging elements, and drive mechanisms for moving the optical components. The measurement optical system 36 allows the examiner to observe and image the anterior ocular segment, cornea, fundus, and other structures of the subject eye E of the examinee P.

The monitor portion 32 is attached to the top portion of the measurement head 31. The monitor portion 32 is formed of a liquid crystal display and includes a touch-panel display screen that displays various items such as the subject eye image and operation buttons. The examiner operates the display screen (operation portion) of the monitor portion 32 to control the movement of the measurement head 31. The monitor portion 32 displays images on the display screen under the control of the controller 35 and sends electrical signals to the controller 35 based on the examiner's interactions with the display.

The monitor portion 32 is attached to a supporting portion 32a, which is fixed to one side of the top portion of the measurement head 31, allowing it to rotate about both a horizontal axis (X axis or Z axis) and a vertical axis (Y axis). This configuration enables the examiner to adjust the angle and direction of the display screen of the monitor portion 32 to suit the examiner's position, posture, eye level, and other relevant factors.

The forehead rest 33 is provided on the pedestal 30. The forehead rest 33 includes a pair of columnar portions 33a projecting upward from both sides of the pedestal 30, and a forehead rest body 33b connected to the upper ends of the columnar portions 33a.

The chin rest device 18E in the sixth embodiment is detachably attached to the pedestal 30. The chin rest device 18E includes an attachment 181, a chin rest portion 182, and a vertical-position adjusting mechanism 183. In this embodiment, the chin rest portion 182 includes a housing 182b that is elongated in the vertical direction, and a chin receiving platform 182c that extends from the housing 182b and is movable in the vertical direction. The attachment 181 is provided at the rear side (side facing the body portion 10E) of the lower end of the housing 182b. The attachment 181 includes, for example, one or more magnets provided in the housing 182b. The one or more magnets are attracted to a metal part of the pedestal 30 or to one or more magnets provided on the pedestal 30, thereby attaching the chin rest device 18E to the pedestal 30. Alternatively, the attachment 181 may include one or more projections provided on the housing 182b, and the projection may engage with one or more receivers provided on the pedestal 30, securing the chin rest device 18E to the pedestal 30.

The vertical-position adjusting mechanism 183 of the sixth embodiment is housed within the housing 182b. The vertical-position adjusting mechanism 183 includes an electric drive portion 183d, which includes an electric motor, an encoder, and other related components, as well as a cable 183e, similar to the configuration in the fifth embodiment. A terminal 183f at the end of the cable 183e is connected to a plug-in port 30a on the pedestal 30, which is, in turn, connected to the controller 35 via a cable not shown in the drawings. The cable 183e transmits instruction signals from the controller 35 and electric power to the electric drive portion 183d.

The attachment 181 of the chin rest device 18E in the sixth embodiment may have the same configuration as any of the chin rest devices 18 to 18D of the first to fifth embodiments. Furthermore, a chin rest device having the same configuration as any one of the chin rest devices 18 to 18D of the first to fifth embodiments may be detachably attached to the pedestal 30 of the ophthalmologic apparatus 100E in the sixth embodiment. Alternatively, any one of the chin rest devices 18 to 18D of the first to fifth embodiments may be attached to the optical table 12E, which supports the ophthalmologic apparatus 100E in the sixth embodiment.

The examinee P, who faces the ophthalmologic apparatus 100E of the sixth embodiment with the above configuration, applies his or her forehead to the forehead rest body 33b of the forehead rest 33. The examiner vertically moves the optical table 12 to adjust the position in the Y direction, as necessary. Next, the examiner operates the operation screen displayed on the display screen of the monitor portion 32 to adjust the height of the chin rest device 18E. The controller 35 controls the electric drive portion 183d to move the chin receiving platform 182c vertically based on the input operation. This positions the chin receiving platform 182c to receive the chin C of the examinee P, thereby supporting the chin C with the chin receiving platform 182c. Accordingly, the ophthalmologic apparatus 100E of the sixth embodiment can also stably fix the face of the examinee P with both the forehead rest 33 and the chin rest device 18E, allowing the examinee P to undergo the examinations more comfortably in a comfortable posture, thereby enabling more accurate and efficient objective and subjective examinations.

As described above, the chin rest devices 18, 18A, 18B, 18C, and 18D of the first to fifth embodiments are detachably attached to the optical table 12 of the ophthalmologic apparatus 100, which acquires information about the subject eyes E. Each of the chin rest devices 18 to 18D includes the attachment 181 that is detachably attached to the optical table 12, and the chin rest portion 182 that extends from the attachment 181 toward the chin C of the examinee P to support the chin C. The ophthalmologic apparatus 100 in each of the embodiments includes the measurement heads 16 with the measurement optical systems 21, 21 for acquiring information about the subject eyes E, the optical table 12 supporting the measurement heads 16, and any of the chin rest devices 18 to 18D. These chin rest devices 18 to 18D are each detachably attached to the optical table 12.

The chin rest device 18E according to the sixth embodiment is detachably attached to the pedestal 30, which is included in the ophthalmologic apparatus 100E for acquiring information about the subject eyes E. The measurement head 31 is supported on the pedestal 30, allowing movement in both horizontal and vertical directions. The chin rest device 18E includes the attachment 181 that is detachably attached to the pedestal 30, and the chin rest portion 182 that extends from the attachment 181 toward the chin C of the examinee P to support the chin C. The ophthalmologic apparatus 100E of the sixth embodiment may include the pedestal 30, the measurement head 16 that includes the measurement optical system 21 for acquiring information about the subject eyes E and is supported on the pedestal 30 to be movable vertically relative to the subject eyes E, and any of the chin rest devices 18 to 18E. These chin rest devices 18 to 18E may each be detachably attached to the pedestal 30.

Accordingly, each of the chin rest devices 18 to 18E of the first to sixth embodiments can stably fix the face of the examinee P, preventing the accidental movement of the face. Thus, the ophthalmologic apparatus 100, 100E of the embodiments including any of the chin rest devices 18 to 18E can provide more stable support for the face of the examinee P, allowing more appropriate and efficient acquisition of information about the subject eyes E more appropriately and efficiently. As a result, it is possible to acquire information about the subject eyes E more comfortably for the examinee P and more accurately.

The chin rest devices 18A and 18B of the second and third embodiments each include the retraction mechanism 184 that retracts the chin rest portion 182 to the predetermined retraction position W of the optical table 12. This allows the ophthalmologic apparatus 100 of the second and third embodiments to perform examinations with the option of using or not using the chin rest portion 182. When the chin rest portion 182 is retracted to the retraction position W during non-use, more free space becomes available on the top surface of the optical table 12. This makes it easier for the examinee P to rest his or her hands or arms on the optical table 12, and to place examination equipment, the examinee's control device, and/or other items on it. Furthermore, during the examination such as the subjective examination, the examinee P is not restricted in moving his or her chin C and can respond verbally more smoothly, thereby making the examinations more comfortable and effective.

In each of the embodiments, the attachment 181 includes the upper support member 181a that is disposed on the upper surface of the optical table 12 (or pedestal 30), the lower support member 181b that is disposed on the lower surface of the optical table 12, the connecting portion 181c that connects one sides of the upper and lower support members 181a and 181b, and the attachment space 181d that is defined by the upper and lower support members 181a and 181b, as well as the connecting portion 181c to receive the optical table 12 (or pedestal 30). This configuration allows the user to easily attach and detach each of the chin rest devices 18 to 18E to and from the optical table 12 (or pedestal 30) by inserting or removing the optical table 12 (or pedestal 30) into or out of the attachment space 181d. Clamping the optical table 12 (or pedestal 30) between the upper support member 181a and the lower support member 181b ensures a stable attachment of each chin rest device 18 to 18E to the optical table 12 (or pedestal 30).

Each chin rest device 18 to 18E in the above embodiments includes the vertical-position adjusting mechanism 183 that can adjust the position of the chin rest portion 182 in the vertical direction. This configuration allows the position of the chin rest portion 182 to be adjusted vertically according to the position of the chin C of the examinee P, enabling the chin rest portion 182 to support the chin C more stably.

Each ophthalmologic apparatus 100 in the first to fifth embodiments includes the pair of measurement heads 16, the head supporting portion 13, and the drive mechanisms 15. The pair of measurement heads 16 is positioned on the left and right sides to correspond to the left and right subject eyes E, respectively. Each of the measurement heads 16 includes the measurement optical system 21, the image acquiring portion that captures the anterior-ocular-segment images of the subject eye E along the optical axis of the measurement optical system, and the visual-target presenting portion that presents the visual targets to the subject eye E. The head supporting portion 13 is provided on the optical table 12 and individually suspends the measurement heads 16 to support them. Each of the drive mechanisms 15 moves the corresponding measurement head 16 in the vertical and horizontal directions and rotates the measurement head 16 about a rotation axis parallel to the vertical direction and another rotation axis parallel to the horizontal direction. Each of the measurement heads 16 is suspended from the head supporting portion 13 via the drive mechanism 15.

This configuration does not position the body portion 10 in front of the examinee P (more specifically, on the optical table 12), thereby creating free space in that area. As a result, the examinee P can undergo the examinations in a comfortable posture without being obstructed by the body portion 10. Furthermore, the examinee P can participate in the examinations more stably and efficiently by supporting his or her body with hands placed on the optical table 12.

The ophthalmologic apparatus 100 in the first to fifth embodiments can acquire information about the subject eyes E simultaneously for both subject eyes or individually for each of the subject eyes E using a pair of measurement heads 16. Additionally, the measurement heads 16 can be freely moved by the drive mechanism 15, allowing for more detailed and accurate information acquisition regarding the subject eyes E. Furthermore, each chin rest device 18 to 18D, which is attached to the optical table 12, remains unaffected by the movement of the measurement heads 16, thereby maintaining stable support for the chin C.

The embodiments and modified embodiments of the present invention regarding the ophthalmologic apparatus have been described above. The specific configurations are not limited to these embodiments and modifications. Design changes, additions, and alterations are permitted as long as they do not deviate from the gist of the inventions recited in the claims.

The ophthalmologic apparatus of a modified embodiment may include a measurement head including a measurement optical system with a plurality of examination lenses selectively positioned in front of the subject eye E, and a visual-target presenting portion that presents a visual target to the subject eye. More specifically, the ophthalmologic apparatus may include a phoropter head and a visual-target presenting portion as described in JP2023-048902A. Each of the chin rest devices 18 to 18D of the first to fifth embodiments can be detachably attached to the optical table of such an ophthalmologic apparatus. As a result, the ophthalmologic apparatus of the modified embodiment supports the face of the examinee P more stably, allowing the examinee P to undergo the examinations in a comfortable posture and enabling more accurate and efficient acquisition of information about the subject eyes E.

The ophthalmologic apparatus 100 of each of the embodiments and modified embodiments includes a pair of measurement heads 16. However, it is not limited to this configuration. The ophthalmologic apparatus may also include only one measurement head 16, allowing for the acquisition of the ocular information for one eye at a time.

Regarding the above description of the first to fifth embodiments and the modified embodiment, the following is disclosed.
(1) A chin rest device that is detachably attached to an optical table of an ophthalmologic apparatus, the optical table configured to acquire information about a subject eye, the chin rest device including:
   an attachment that is configured to be detachably attached to the optical table; and
   a chin rest portion that is configured to extend from the attachment toward a chin of an examinee to support the chin.
(2) The chin rest device according to the above (1), further including a retraction mechanism that is configured to retract the chin rest portion to a predetermined retraction position of the optical table.
(3) The chin rest device according to the above (1) or (2), wherein the attachment includes:
   an upper support member that is disposed on an upper surface of the optical table;
   a lower support member that is disposed on a lower surface of the optical table;
   a connecting portion that connects one side of the upper support member and one side of the lower support member; and
   an attachment space that is defined by the upper and lower support members and the connecting portion, the attachment space being configured to receive the optical table.
(4) The chin rest device according to any one of the above (1) to (3), further including a vertical-position adjusting mechanism that is configured to adjust a vertical position of the chin rest portion.
(5) An ophthalmologic apparatus, including:
   a measurement head including a measurement optical system that is configured to acquire information about a subject eye;
   an optical table that is configured to support the measurement head; and
   the chin rest device according to any one of the above (1) to (4),
   wherein the chin rest device is configured to be detachably attached to the optical table.
(6) The ophthalmologic apparatus according to the above (5), wherein the ophthalmologic apparatus includes:
   a pair of measurement heads on left and right sides corresponding to left and right subject eyes, each of the measurement heads including:
      the measurement optical system;
      an image acquiring portion that is configured to acquire an anterior-ocular-segment image on an optical axis of the measurement optical system of the subject eye; and
      a visual-target presenting portion that is configured to present a visual target to the subject eye;
   a head supporting portion that is provided on the optical table to support the pair of measurement heads by separately suspending the pair of measurement heads; and
   a drive mechanism that is configured to move each of the measurement heads in vertical and horizontal directions and to rotate each of the measurement heads about a rotational axis parallel to the vertical direction and a rotational axis parallel to the horizontal direction,
   wherein each of the measurement heads is configured to be suspended from the head supporting portion via the drive mechanism.
(7) The ophthalmologic apparatus according to the above (5),
   wherein the measurement head including the measurement optical system including a plurality of examination lenses that are selectively disposed in front of the subject eye; and
   wherein the ophthalmologic apparatus further includes a visual-target presenting portion that is configured to present a visual target to the subject eye.

The present invention further discloses the following.
(1) A chin rest device that is detachably attached to a pedestal of an ophthalmologic apparatus that is configured to acquire information about a subject eye, the pedestal configured
   to support a measurement head for acquiring information about the subject eye such that the measurement head is movable in horizontal and vertical directions,
   the chin rest device including:
   an attachment that is configured to be detachably attached to the pedestal; and
   a chin rest portion that is configured to extend from the attachment toward a chin of an examinee to support the chin.
(2) The chin rest device according to the above (1), further including a retraction mechanism that is configured to retract the chin rest portion to a predetermined retraction position of the pedestal.
(3) The chin rest device according to the above (1) or (2), wherein the attachment includes:
   an upper support member that is disposed on an upper surface of the pedestal;
   a lower support member that is disposed on a lower surface of the pedestal;
   a connecting portion that connects one side of the upper support member and one side of the lower support member; and
   an attachment space that is defined by the upper and lower support members and the connecting portion, the attachment space being configured to receive the pedestal.
(4) The chin rest device according to any one of the above (1) to (3), further including a vertical-position adjusting mechanism that is configured to adjust a vertical position of the chin rest portion.
(5) An ophthalmologic apparatus, including:
   a pedestal; and
   a measurement head including a measurement optical system that is configured to acquire information about a subject eye, the measurement head being supported on the pedestal such that the measurement head is movable relative to the subject eye in a horizontal direction; and
   the chin rest device according to any one of the above (1) to (4),
   wherein the chin rest device is configured to be detachably attached to the pedestal.

### List of Reference Signs

12 Optical table
13 Head supporting portion
15 Drive mechanism
16, 31 Measurement head
18, 18A, 18B, 18C, 18D, 18E Chin rest device
21, 36 Measurement optical system
30 Pedestal
100, 100E Ophthalmologic apparatus
181 Attachment
181a Upper support member
181b Lower support member
181c Connecting portion
181d Attachment space
182 Chin rest portion
183 Vertical-position adjusting mechanism
184 Retraction mechanism
C Chin
E Subject eye
P Examinee
W Retraction position

## Claims

1. A chin rest device (18, 18A, 18B, 18C, 18D, 18E),
wherein the chin rest device (18, 18A, 18B, 18C, 18D, 18E) is configured to be detachably attached to an optical table (12) or a pedestal (30), the optical table (12) of an ophthalmologic apparatus (100) is configured to acquire information about a subject eye (E), and the pedestal (30) is configured to support a measurement head (16, 31) for acquiring information about the subject eye (E) such that the measurement head (16, 31) is movable in horizontal and vertical directions,
wherein the chin rest device (18, 18A, 18B, 18C, 18D, 18E) comprises:
an attachment (181) that is configured to be detachably attached to the optical table (12) or the pedestal (30); and
a chin rest portion (182) that is configured to extend from the attachment (181) toward a chin (C) of an examinee (P) to support the chin (C).

2. The chin rest device (18, 18A, 18B, 18C, 18D, 18E) according to claim 1, further comprising a retraction mechanism (184) that is configured to retract the chin rest portion (182) to a predetermined retraction position (W) of the optical table (12) or the pedestal (30).

3. The chin rest device (18, 18A, 18B, 18C, 18D, 18E) according to claim 1, wherein the attachment (181) comprises:
an upper support member (181a) that is disposed on an upper surface of the optical table (12) or the pedestal (30);
a lower support member (181b) that is disposed on a lower surface of the optical table (12) or the pedestal (30);
a connecting portion (181c) that connects one side of the upper support member (181a) and one side of the lower support member (181b); and
an attachment space (181d) that is defined by the upper and lower support members (181a, 181b) and the connecting portion (181c), the attachment space (181d) being configured to receive the optical table (12) or the pedestal (30).

4. The chin rest device (18, 18A, 18B, 18C, 18D, 18E) according to claim 1, further comprising a vertical-position adjusting mechanism (183) that is configured to adjust a vertical position of the chin rest portion (182).

5. An ophthalmologic apparatus (100), comprising:
a measurement head (16) comprising a measurement optical system (21) that is configured to acquire information about a subject eye (E);
an optical table (12) that is configured to support the measurement head (16); and
the chin rest device (18, 18A, 18B, 18C, 18D) according to any one of claims 1 to 4,
wherein the chin rest device (18, 18A, 18B, 18C, 18D) is configured to be detachably attached to the optical table (12).

6. The ophthalmologic apparatus (100) according to claim 5, wherein the ophthalmologic apparatus (100) comprises:
a pair of measurement heads (16) on left and right sides corresponding to left and right subject eyes (E), each of the measurement heads (16) comprising:
the measurement optical system (21);
an image acquiring portion that is configured to acquire an anterior-ocular-segment image on an optical axis of the measurement optical system (21) of the subject eye (E); and
a visual-target presenting portion that is configured to present a visual target to the subject eye (E);
a head supporting portion (13) that is provided on the optical table (12) to support the pair of measurement heads (16) by separately suspending the pair of measurement heads (16); and
a drive mechanism (15) that is configured to move each of the measurement heads (16) in vertical and horizontal directions and to rotate each of the measurement heads (16) about a rotational axis parallel to the vertical direction and a rotational axis parallel to the horizontal direction,
wherein each of the measurement heads (16) is configured to be suspended from the head supporting portion (13) via the drive mechanism (15).

7. The ophthalmologic apparatus (100) according to claim 5,
wherein the measurement head (16) comprises the measurement optical system (21) comprising a plurality of examination lenses that are selectively disposed in front of the subject eye (E), and
wherein the ophthalmologic apparatus (100) further comprises a visual-target presenting portion that is configured to present a visual target to the subject eye (E).

8. An ophthalmologic apparatus (100E), comprising:
a pedestal (30);
a measurement head (31) comprising a measurement optical system (36) that is configured to acquire information about a subject eye (E), the measurement head (31) being supported on the pedestal (30) such that the measurement head (31) is movable relative to the subject eye (E) in horizontal and vertical directions; and
the chin rest device (18E) according to any one of claims 1 to 4,
wherein the chin rest device (18E) is configured to be detachably attached to the pedestal (30).
